Europäisches Patentamt

European Patent Office

Office européen des brevets

(11) Publication number: **0 082 702**
**B1**

(12) EUROPEAN PATENT SPECIFICATION

(45) Date of publication of patent specification: **27.08.86**

(21) Application number: **82306781.4**

(22) Date of filing: **20.12.82**

(51) Int. Cl.⁴: **C 07 C 15/04,** C 07 C 15/06,
C 07 C 15/08, C 07 C 1/20

(54) Conversion of Fischer-Tropsch synthesis products to benzene, xylene and toluene.

(30) Priority: **23.12.81 US 333845**

(43) Date of publication of application:
**29.06.83 Bulletin 83/26**

(45) Publication of the grant of the patent:
**27.08.86 Bulletin 86/35**

(84) Designated Contracting States:
**BE DE FR GB IT NL**

(56) References cited:
**DE-A-3 113 838**
**GB-A-1 446 252**
**US-A-4 076 761**

(73) Proprietor: **MOBIL OIL CORPORATION**
**150 East 42nd Street**
**New York New York 10017 (US)**

(72) Inventor: **Chu, Yung-Feng**
**121 Randle Drive**
**Cherry Hill New Jersey 08034 (US)**
Inventor: **Chester, Arthur Warren**
**517 Country Club Drive**
**Cherry Hill New Jersey 08003 (US)**

(74) Representative: **West, Alan Harry et al**
**Mobil Court 3 Clements Inn**
**London WC2A 2EB (GB)**

Courier Press, Leamington Spa, England.

## Description

This invention relates to a process for converting oxygenated products derived from Fischer-Tropsch synthesis products to benzene, toluene, and xylene for petrochemical feedstocks.

Processes for the conversion of coal and other hydrocarbons such as natural gas to a gaseous mixture consisting essentially of hydrogen and carbon monoxide, or of hydrogen and carbon dioxide, or of hydrogen and carbon monoxide and carbon dioxide, are well known. Although various processes may be employed for the gasification, those of major importance depend either on the partial combustion of the fuel with an oxygen-containing gas or on a combination of these two reactions. A summary of the art of gas manufacture, including synthesis gas, from solid and liquid fuels, is given in Encyclopedia of Chemical Technology, Edited by Kirk-Othmer, Second Edition, Volume 10, pages 353—433, (1966), Interscience Publishers, New York, N.Y., the contents of which are herein incorporated by reference. Volume 4, pp. 446—488, Interscience Publishers, New York, N.Y.

It is desirable to convert synthesis gas, obtained from coal, natural gas or any other available source to highly valued hydrocarbons such as gasoline with relatively high octane number, petrochemical feedstocks, liquefiable petroleum fuel gas, and aromatic hydrocarbons. It is well known that synthesis gas will undergo conversion to form reduction products of carbon monoxide, such as oxygenates and hydrocarbons, at temperatures in the range of from 300°F to 850°F (149°C to 454°C) under pressures of from about one to one thousand atmospheres pressure, over a fairly wide selection of catalyst compositions. The Fischer-Tropsch process, for example, which has been most extensively studied, produces a range of products including oxygenates, heavy waxy oils, and liquid hydrocarbons which have been used as low octane gasoline. The types of catalyst that have been studied for this process include those based on metals or oxides of iron, cobalt, nickel, ruthenium, thorium, rhodium and osmium.

In addition, British Patent No. 1,446,522 discloses a method of producing aromatic hydrocarbons in which synthesis gas is converted to oxygenates, conveniently by the Fischer/Tropsch reaction, and the resultant oxygenates are contacted with a catalyst comprising a crystalline alumina-silicate zeolite having a silica/alumina ratio of at least 12 and a constraint index between 1 and 12. The zeolite may be in the hydrogen form or may be base exchanged with a metal from one or more of Groups IB, IIA, IIB, IIA, IIIB, IVA, IVB, and VIII of the periodic table. It has, however, now been found that the process disclosed in British Patent No. 1,446,522 is particularly effective to produce benzene, toluene and xylene if the zeolite is promoted with gallium.

Accordingly, the present invention resides in a process for producing a mixture of hydrocarbons comprising

(a) converting a fossil fuel to a synthesis gas;

(b) converting the synthesis gas by Fischer-Tropsch reaction to a product containing oxygenates and hydrocarbons; and

(c) contacting said Fischer-Tropsch product with a catalyst comprising a metal-promoted porous crystalline zeolite having a constraint index within the range of 1 to 12 and a silica to alumina ratio of at least 12,

characterized in that the metal promoter is gallium and benzene, toluene and xylene and removed from the effluent stream of step (c).

The gallium promoter in the zeolite catalyst may be present as gallium oxide and/or as gallium ions if cations in the ZSM-5 type zeolite have been exchanged with gallium ions therein.

Such catalysts may be produced by conventional ion exchange techniques and the catalysts so produced subsequently dried. For example, an aqueous solution of gallium nitrate may be placed in contact with the zeolite at ambient or elevated temperature, e.g. by refluxing. The exchanged zeolite is then separated by decantation followed by filtration, washed several times with deionized water and finally dried. Before addition to the aqueous solution of the gallium compound, the zeolite may be acid treated.

The process of the present invention may also be carried out using catalysts in which the gallium is only impregnated on the surface of the zeolite or is incorporated in the intra-crystalline zeolite cavities as a gallium compound which gives rise to gallium oxide during activation of the catalyst prior to contact with the hydrocarbon feedstock.

Where the catalyst composition is prepared by using a gallium compound which ionizes in aqueous solution, for example, gallium nitrate, it is inevitable that some of the gallium ions will be exchanged with the cations in the zeolite even if the preparation was directed to impregnation of the zeolite.

Whichever method of catalyst preparation is used, the amount of gallium present in the catalyst compositions (metal plus zeolite) may suitably vary between 0.01 and 5 percent, preferably between 0.05 and 2 percent by weight.

The ZSM-5 type crystalline zeolites utilized herein are members of a class of zeolitic materials which exhibit unusual properties. Although these zeolites have unusually low alumina contents, i.e. high silica to alumina mole ratios, they are very active even when the silica to alumina mole ratio exceeds 30. The activity is surprising since catalytic activity is generally attributed to framework aluminum atoms and/or cations associated with these aluminum atoms. These zeolites retain their crystallinity for long periods in spite of the presence of steam at high temperature which induces irreversible collapse of the framework of other zeolites, e.g. of the X and A type. Furthermore, carbonaceous deposits, when formed, may be removed by burning at higher than usual temperatures to restore activity. These zeolites, used as catalysts, generally have low coke-forming activity and therefore are conducive to long times on stream between regenerations by burning carbonaceous deposits with oxygen-containing gas such as air.

An important characteristic of the crystal structure of this class of zeolites is that it provides a selective constrained access to and egress from the intracrystalline free space by virtue of having an effective pore size intermediate between the small pore Linde A and the large pore Linde X, i.e. the pore windows of the structure are of about a size such as would be provided by 10-membered rings of silicon atoms interconnected by oxygen atoms. It is to be understood, of course, that these rings are those formed by the regular disposition of the tetrahedra making up the anionic framework of the crystalline zeolite, the oxygen atoms themselves being bonded to the silicon (or aluminum, etc.) atoms at the centers of the tetrahedra.

The silica to alumina mole ratio referred to may be determined by conventional analysis. This ratio is meant to represent, as closely as possible, the ratio in the rigid anionic framework of the zeolite crystal and to exclude aluminum in the binder or in cationic or other form within the channels. Although zeolites with silica to alumina mole ratios of at least 12 are useful, it is preferred in some instances to use zeolites having substantially higher silica/alumina ratios, e.g. 1600 and above. In addition, zeolites as otherwise characterized herein but which are substantially free of aluminum, that is zeolites having silica to alumina mole ratios of up to infinity, are found to be useful and even preferable in some instances. Such "high silica" or "highly siliceous" zeolites are intended to be included within this description. Also included within this definition are substantially pure silica analogs of the useful zeolites described herein, that is to say those zeolites having no measurable amount of aluminum (silica to alumina mole ratio of infinity) but which otherwise embody the characteristics disclosed.

These zeolites, after activation, acquire an intracrystalline sorption capacity for normal hexane which is greater than that for water, i.e. they exhibit "hydrophobic" properties. This hydrophobic character can be used to advantage in some applications.

The class of zeolites useful herein have an effective pore size such as to freely sorb normal hexane. In addition, the structure must provide constrained access to larger molecules. It is sometimes possible to judge from a known crystal structure whether such constrained access exists. For example, if the only pore windows in a crystal are formed by 8-membered rings of silicon and aluminum atoms, then access by molecules of larger cross-section than normal hexane is excluded and the zeolite is not of the desired type. Windows of 10-membered rings are preferred, although in some instances excessive puckering of the rings or pore blockage may render these zeolites ineffective.

Although 12-membered rings in theory would not offer sufficient constraint to produce advantageous conversions, it is noted that the puckered 12-ring structure of TMA offretite does show some constrained access. Other 12-ring structures may exist which may be operative for other reasons and, therefore, it is not the present intension to entirely judge the usefulness of a particular zeolite solely from theoretical structural considerations.

Rather than attempt to judge from crystal structure whether or not a zeolite possesses the necessary constrained access to molecules of larger cross-section than normal paraffins, a simple determination of the "Constraint Index" may be used. The meaning of Constraint Index and its method of determination are fully described in, for example, U.S. Patent No. 3,905,915.

Preferably the zeolite used herein is selected from ZSM-5, ZSM-11, ZSM-12, ZSM-23, ZSM-35, ZSM-38, ZSM-48, and other similar materials, with ZSM-5 being especially preferred.

ZSM-5 is described in greater detail in U.S. Patents No. 3,702,886 and Re 29,948. ZSM-11 is described in U.S. Patent No. 3,709,979. ZSM-12 is described in U.S. Patent No. 3,832,449. ZSM-23 is described in U.S. Patent No. 4,076,842. ZSM-35 is decribed in U.S. Patent No. 4,016,245. ZSM-38 is described in U.S. Patent No. 4,046,859. ZSM-48 is described in published European Patent Application No. 15132.

The specific zeolites described, when prepared in the presence of organic cations, are substantially catalytically inactive, possibly because the intra-crystalline free space is occupied by organic cations from the forming solution. They may be activated by heating in an inert atmosphere at 540°C for one hour, for example, followed by base exchange with ammonium salts followed by calcination at 540°C in air. The presence of organic cations in the forming solution may not be absolutely essential to the formation of this type zeolite; however, the presence of these cations does appear to favor the formation of this class of zeolite. More generally, it is desirable to activate this type catalyst by base exchange with ammonium salts followed by calcination in air at about 540°C for from 15 minutes to 24 hours.

Natural zeolites may sometimes be converted to zeolite structures of the class herein identified by various activation procedures and other treatments such as base exchange, steaming, alumina extraction and calcination, alone or in combinations. Natural minerals which may be so treated include ferrierite, brewsterite, stilbite, dachiardite, epistilbite, heulandite, and clinoptilolite.

In a preferred aspect of this invention, the zeolites hereof are selected as those providing among other things a crystal framework density, in the dry hydrogen form, of not less than 1.6 grams per cubic centimeter. The dry density for known structures may be calculated from the number of silicon plus aluminum atoms per 1000 cubic Angstroms, as given, e.g., on Page 19 of the article Zeolite Structure by W. M. Meier in Proceedings of the Conference on Molecular sieves, (London, April 1967) published by the Society of Chemical Industry, London, 1968.

When the crystal structure is unknown, the crystal framework density may be determined by classical pyknometer techniques. For example, it may be determined by immersing the dry hydrogen form of the zeolite in an organic solvent which is not sorbed by the crystal. Or, the crystal density may be determined by mercury porosimetry, since mercury will fill the interstices between crystals but will not penetrate the intracrystalline free space.

When synthesized in the alkali metal form, the zeolite is conveniently converted to the hydrogen form, generally by intermediate formation of the ammonium form as a result of ammonium ion exchange and calcination of the ammonium form to yield the hydrogen form. In addition to the hydrogen form, other forms of the zeolite wherein the original alkali metal has been reduced to less than about 1.5 percent by weight may be used. Thus, the original alkali metal of the zeolite may be replaced by ion exchange with other suitable metal cations of Groups I through VIII of the Periodic Table, including, by way of example, nickel, copper, zinc, palladium, calcium or rare earth metals.

In practicing a particularly desired chemical conversion process, it may be useful to incorporate the above-described crystalline zeolite with a matrix comprising another material resistant to the temperature and other conditions employed in the process. Such matrix material is useful as a binder and imparts greater resistance to the catalyst for the severe temperature, pressure and reactant feed stream velocity conditions encountered in many cracking processes.

Useful matrix materials include both synthetic and naturally occurring substances, as well as inorganic materials such as clay, silica and/or metal oxides. The latter may be either naturally occurring or in the form of gelatinous precipitates or gels including mixtures of silica and metal oxides. Naturally occurring clays which can be composited with the zeolite include those of the montmorillonite and kaolin families, which families include the sub-bentonites and the kaolins commonly known as Dixie, McNamee-Georgia and Florida clays or others in which the main mineral constituent is halloysite, kaolinite, dickite, nacrite or anauxite. Such clays can be used in the raw state as originally mined or initially subjected to calcination, acid treatment or chemical modification.

In addition to the foregoing materials, the zeolites employed herein may be composited with a porous matrix material, such as alumina, silica-alumina, silica-magnesia, silica-zirconia, silica-thoria, silica-beryllia, and silica-titania, as well as ternary compositions, such as silica-alumina-thoria, silica-alumina-zirconia, silica-alumina-magnesia and silica-magnesia-zirconia. The matrix may be in the form of a cogel. The relative proportions of zeolite component and inorganic oxide gel matrix, on an anhydrous basis, may vary widely with the zeolite content ranging from between 1 to 99 percent by weight and more usually in the range of 5 to 80 percent by weight of the dry composite.

A Fischer-Tropsch Synthesis gas conversion process is relied upon to convert syngas obtained from coal to form particularly hydrocarbons, oxygenates and chemical forming components. The process is complex, expensive to operate and may be conveniently divided into (1) a complex for preparing synthesis gas from coal, (2) a Fischer-Tropsch type of synthesis gas conversion using a fixed catalyst bed operation, a fluid catalyst bed operation, or a slurry bed operation, (3) a product recovery operation, and (4) an auxiliary plant and utility operations required in such a complex.

The extremely diverse nature of the products obtained in such a combination operation amplifies the complexity of the overall process arrangement and its operating economics. The synthesis operation is known to produce a wide spectrum of products including fuel gas, light olefins, LPG, gasoline, light and heavy fuel oils, waxy oils and oxygenates identified as alcohol, acetones, ketones, and acids, particularly acetic and proprionic acid. The $C_2$ and lower boiling components may be reformed to carbon monoxide and hydrogen or the $C_2$ formed hydrocarbons and methane may be combined and blended for use in a fuel gas pipeline system.

In the operation, the water soluble oxygenates or chemicals are separated and recovered as individual components with the formed organic acids remaining in the water phase separately processed. Disposal of the oxygenates and formed acids is a very expensive operation. Propylene and butylene formed in the process are converted to gasoline boiling components by polymerization in the presence of a solid phosphoric acid catalyst. Propane and butane on the other hand are used for LPG.

The present invention is concerned with converting the oxygenate and hydrocarbon product of a Fischer-Tropsch synthesis gas conversion operation to premium chemical feedstocks i.e. benzene, toluene and xylene.

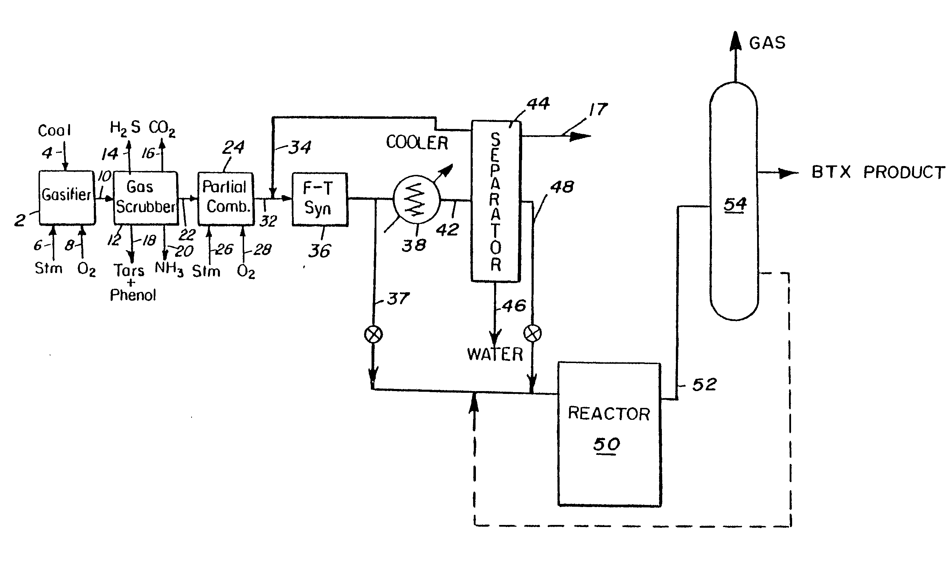

The accompanying drawing is a condensed schematic block flow arrangement of a process directed to the conversion of coal to synthesis gas comprising carbon monoxide and hydrogen, the reduction of carbon monoxide by the Fischer-Tropsch process to form a product mixture comprising hydrocarbons and oxygenates and the recovery of these products for further use.

Referring to the drawing, a coal gasifier section 2 is supplied with pulverised coal by way of a conduit 4 with steam by way of a conduit 6 and with oxygen by way of a conduit 8. The products of gasifier section 2 are then fed through conduit 10 to a gas scrubber section 12. In scrubber section 12, carbon monoxide and hydrogen are separated from hydrogen sulfide which is removed through conduit 14. From carbon dioxide which is removed through conduit 16, from tars and phenols which are removed through conduit 18 and from ammonia which is removed through conduit 20. The carbon monoxide-hydrogen rich gas is passed from section 12 by conduit 22 to a partial combustion zone 24 supplied with steam by conduit 26 and oxygen by conduit 28. The partial combustion operation of section 24 produces a carbon monoxide-hydrogen synthesis gas of desired ratio which is then passed by conduit 32 to a Fischer-Tropsch reaction section 36 which is provided with for example an iron synthesis catalyst and which also receives a $C_2$ recycle gas introduced by conduit 34. Generally, the synthesis gas feed is introduced into the section 36 at a temperature of about 160°C and at an elevated pressure of about 365 psig. However, the temperature of the synthesis gas admixed with catalyst to the fluid operation rapidly rises because of the heat liberated so that the Fischer-Tropsch and water gas shift reactions are conveyed to a cooler, 38, and the cooled products are then conveyed via line 42 to a separator 44. In practice the cooler 38, line 42 and separator 44 may be one integral unit. Water is removed from separator 44 via line 46, $C_2$ recycled by conduit 34, other gases via line 17, and liquid hydrocarbon products via line 48. The liquid product is conveyed via line 48 to reactor 50 where it is contacted with the metal impregnated crystalline zeolite.

The flow stream from either Fischer-Tropsch reaction section 36 through line 37 or from separator 44 through line 48 is contacted with the ZSM-5 type zeolite in the reactor 50, preferably in the form of a fixed bed. In a typical, and preferred embodiment of the process of this invention, the feedstream of oxygenates and hydrocarbons is introduced into the reactor 50 at a temperature within the range of 250°C to 650°C, a pressure within the range of $1 \times 10^5$ to $30 \times 10^5$ pascal (0 to 400 psig), and a WHSV of 0.1 to 10. Preferred temperatures in the reaction section 36 fall within the range of 400°C to 650°C and preferred pressures fall within the range of $1 \times 10^5$ to $15 \times 10^5$ pascal (0 to 200 psig). A preferred WHSV is between 0.2 and 3.

Effluent from the reactor 50 is conveyed through line 52 optionally to a cooler and separator (not shown) and the liquid portion thereof is fractionated in a distillation tower 54 into fractions consisting of benzene, toluene and xylene. Any fraction containing hydrocarbons in excess of 9 carbon atoms can be recycled through reactor 50 for conversion to benzene, toluene and xylene.

Example 1

Acid or hydrogen-form ZSM-5 zeolite catalyst was impregnated with 0.5% by weight gallium. The catalyst was prepared in the form of extrudate in which the ratio of silica to alumina in the ZSM-5 was 70 to 1. Tests were run using the acid form of zeolite and the acid form containing 0.5% by weight of gallium metal. A Fischer-Tropsch product having the compositions shown in Table I was then passed over each catalyst under the conditions shown in Table I. The results obtained show a substantial increase in the production of aromatics, namely benzenes, toluene and xylene when the gallium impregnated catalyst was used.

In Table II is shown the results obtained with a similar feedstock and catalysts at the same conditions of temperature and pressure as in Table I. In Tables I and II the composition of the feed stock is entered in the column headed "Feed". For the sake of convenience the following abbreviations are used in the Tables:

BTX for benzene, toluene and xylene;
TOS for time on stream;
BZ for benzene; and
EB for ethylbenzene.

TABLE I
BTX from F-T products (100 kPA)

| Catalysts: | | Ga/HZSM-5 | HZSM-5 | Ga/HZSM-5 | HZSM-5 |
|---|---|---|---|---|---|
| Temp., °F | | | 1000 (538°C) | | 1050 (566°C) |
| WHSV | | | 0.5 | | |
| Yield (g/100 g feed) | Feed | | | | |
|    BTX | | 40 | 30 | 41 | 38 |
|    $C_9$+Aromatics | | 15 | 6 | 18 | 8 |
|    Aromatics Total | 1% | 55 | 39 | 59 | 46 |

TABLE II
BTX from Fischer-Tropsch products (100 kPA)

| | 0.5% Ga/HZSM-5 (70/1 $SiO_2/Al_2O_3$) | | | HZSM-5 (70/1 $SiO_2/Al_2O_3$) | | | Feed compositions** | Wt % |
|---|---|---|---|---|---|---|---|---|
| **Catalysts** | | | | | | | | |
| **Conditions:** | | | | | | | | |
| Temp., °F (°C) | 1000 (538) | 1000 (538) | 1050 (566) | 1000 (538) | | 1050 (566) | $C_5$ | 4.8 |
| WHSV | 1 | 0.5 | | | 0.5 | 0.5 | $C_6$ | 8.7 |
| TOS Hrs | 1.5 | 3.0 | 3.5 | 1.0 | | 1.5 | $C_7$ | 10.1 |
| | | | | | | | $C_8$ | 13.1 |
| | | | | | | | $C_9$ | 10.8 |
| **Selectivity, g/100 g Feed** | | | | | | | | |
| Gaseous Product | 44 | 43 | 40 | 56 | | 51 | $C_{10}$ | 9.1 |
| BTX | 36 | 40 | 41 | 33 | | 38 | $C_{11}$ | 7.6 |
| Total Aromatics | 52 | 55 | 59 | 39 | | 46 | $C_{12}$ | 6.5 |
| | | | | | | | $C_{13}$ | 5.3 |
| | | | | | | | $C_{14}$ | 4.4 |
| **Prod. Distribution* Wt%** | | | | | | | | |
| $C_4$- | 44.1 | 43.0 | 39.7 | 56.1 | | 50.7 | $C_{15}$ | 3.9 |
| $C_5$—$C_6$ | 3.5 | 1.6 | 1.2 | 3.5 | | 2.2 | $C_{16}$ | 3.1 |
| BZ | 10.6 | 14.1 | 16.0 | 11.1 | | 14.6 | $C_{17}$ | 2.6 |
| Toluene | 17.9 | 18.7 | 18.7 | 16.2 | | 17.5 | $C_{18}$ | 2.1 |
| EB | 0.7 | 0.7 | 0.8 | 0.7 | | 0.8 | $C_{19}$ | 1.7 |
| Xylenes | 7.7 | 7.5 | 6.6 | 6.0 | | 5.7 | $C_{20}$—$C_{29}$ | 5.2 |
| $C_9$+Aromatics | 15.4 | 14.3 | 17.1 | 6.4 | | 8.4 | $C_{30}$—$C_{40}$ | 1.0 |
| Total | 99.9 | 99.9 | 100.1 | 100.0 | | 99.9 | Total | 100.0 |

* On-line analysis; $H_2$ not analyzed.     ** Contains less than 1% BTX, and approx, 10% oxygenates.

## Claims

1. A process for producing a mixture of hydrocarbons rich in benzene, toluene and xylene comprising:

(a) converting a fossil fuel to a synthesis gas;

(b) converting the synthesis gas by Fischer-Tropsch reaction to a product containing oxygenates and hydrocarbons; and

(c) contacting said Fischer-Tropsch product with a catalyst comprising a metal-promoted porous crystalline zeolite having a constraint index within the range of 1 to 12 and a silica to alumina ratio of at least 12,

characterized in that the metal promoter is gallium and benzene, toluene and xylene are removed from the effluent stream of step (c).

2. The process of Claim 1 characterized in that the contacting step (c) is effected at a temperature of from 250° to 650°C, a pressure of from $1 \times 10^5$ to $30 \times 10^5$ pascal and a WHSV of from 0.1 to 10.

3. The process of any preceding Claim characterized in that said zeolite of step (c) is selected from ZSM-5, ZSM-11, ZSM-12, ZSM-23, ZSM-35, ZSM-38, and ZSM-48.

## Patentansprüche

1. Verfahren zur Herstellung einer Kohlenwasserstoffmischung, die reich an Benzol, Toluol und Xylol ist, welches umfaßt:

(a) Umwandlung eines fossilen Brennstoffes zu Synthesegas;

(b) Umwandlung des Synthesegases durch Fischer-Tropsch-Reaktion zu einem Produkt, das Sauerstoff enthaltende Verbindungen und Kohlenwasserstoffe enthält; und

(c) Inkontaktbringen des Fischer-Tropsch-Produktes mit einem Katalysator, der einen durch Metall beschleunigten porösen, kristallinen Zeolith mit einem Zwangsindex in dem Bereich von 1 bis 12 und einem Siliciumdioxid/Aluminiumoxid-Verhältnis von mindestens 12 umfaßt, dadurch gekennzeichnet, daß der Metallbeschleuniger Gallium ist und Benzol, Toluol und Xylol aus dem Abflußstrom der Stufe (c) entfernt werden.

2. Verfahren nach Anspruch 1, dadurch gekennzeichnet, daß die Stufe des Inkontaktbringens (c) bei einer Temperatur von 250 bis 650°C, einem Druck von $1 \times 10^5$ bis $30 \times 10^5$ Pa und einer WHSV von 0,1 bis 10 durchgeführt wird.

3. Verfahren nach einem der vorstehenden Ansprüche, dadurch gekennzeichnet, daß der Zeolith in Stufe (c) aus ZSM-5, ZSM-11, ZSM-12, ZSM-23, ZSM-35, ZSM-38 und ZSM-48 ausgewählt wird.

## Revendications

1. Un procédé de production d'un mélange d'hydrocarbures riche en benzène, toluène et xylène, comprenant:

(a) la conversion d'un fuel fossile en gaz de synthèse;

(b) la conversion du gaz de synthèse obtenu par la réaction de Fischer-Tropsch en un produit contenant des produits oxygénés et des hydrocarbures; et

(c) la mise au contact dudit produit de la synthèse de Fischer-Tropsch avec un catalyseur comprenant une zéolite cristalline poreuse additionnée d'un métal comme promoteur et ayant un indice de contrainte compris entre 1 et 12 et un rapport silica/alumine d'au moins 12,

caractérisé en ce que le métal promoteur est du gallium et en ce que le benzène, le toluène et le xylène sont enlevès du courant d'effluents de l'étape c.

2. Un procédé selon la revendication 1, caractérisé en ce que l'étape de contact (c) est effectuée à une température de 250°C à 650°C sous une pression de $10^5$ à $30 \times 10^5$ pascal et avec une vitesse spatiale horaire pondérale comprise entre 0,1 et 10.

3. Un procédé selon l'une quelconque des revendications précédentes, caractérisé en ce que ladite zéolite de l'étape (c) est choisie dans le groupe comprenant les ZSM-5, ZSM-11, ZSM-12, ZSM-23, ZSM-35, ZSM-38 et ZSM-48.